(19) Europäisches Patentamt
European Patent Office
Office européen des brevets

(11) Veröffentlichungsnummer: **0 022 446**
**A1**

(12) # EUROPÄISCHE PATENTANMELDUNG

(21) Anmeldenummer: **80101546.2**

(22) Anmeldetag: **24.03.80**

(51) Int. Cl.³: **C 07 D 495/04**, C 07 D 491/044, C 07 D 491/052, C 07 D 311/68 // (C07D495/04, 333/00, 235/00),(C07D491/044, 313/00, 235/00),(C07D491/052, 311/00, 235/00),(C07D491/052, 311/00, 205/00)

(30) Priorität: **06.04.79 CH 3293/79**

(43) Veröffentlichungstag der Anmeldung: **21.01.81 Patentblatt 81/3**

(84) Benannte Vertragsstaaten: **AT BE CH DE FR GB IT NL**

(71) Anmelder: **F. HOFFMANN-LA ROCHE & CO. Aktiengesellschaft, CH-4002 Basel (CH)**

(72) Erfinder: **Hohenlohe-Oehringen, Kraft, Prof. Dr., Schlossfeld 8a, A-6020 Innsbruck (AT)**
Erfinder: **Fliri, Anton, Dr., Lindauerstrasse 61, A-6911 Lochau (AT)**

(74) Vertreter: **Lederer, Franz, Dr. et al, Patentanwälte Dr. Franz Lederer Reiner F. Meyer Lucile-Grahn-Strasse 22, D-8000 München 80 (DE)**

(54) **Imidazolidonderivate, Verfahren zu ihrer Herstellung, ihre Umwandlung in Biotin sowie Ausgangs- und Zwischenprodukte in diesem Verfahren.**

(57) Die Erfindung betrifft ein Verfahren zur Herstellung von Biotin, wobei ausgehend von Verbindungen der Formel I entsprechend der Reaktionsfolge

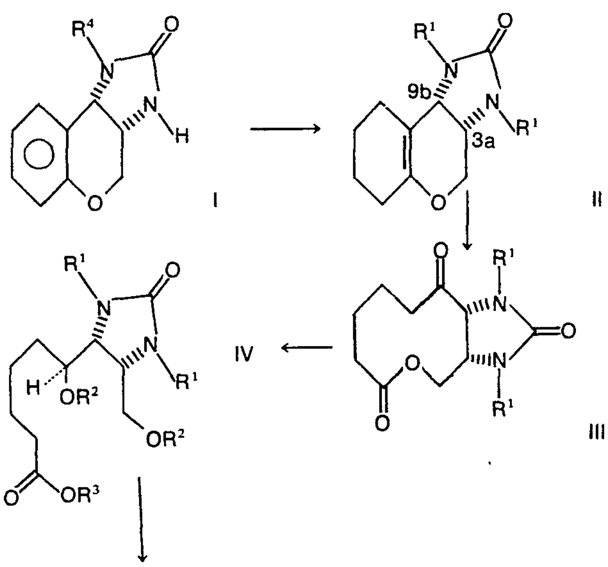

I II III IV

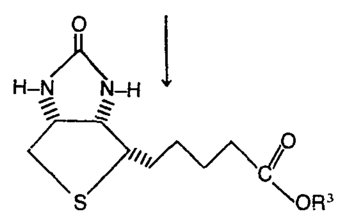

V

wobei R¹, R², R³ und R⁴ die in den Ansprüchen angegebene Bedeutung haben, Biotinester der Formel V erhalten werden, die gewünschtenfalls durch Hydrolyse in Biotin übergeführt werden, sowie Ausgangs- und Zwischenprodukte in diesem Verfahren.

EP 0 022 446 A1

0022446
24. März 1980

F. Hoffmann-La Roche & Co. Aktiengesellschaft, Basel, Schweiz

RAN 4227/50

BEZEICHNUNG GEÄNDERT
siehe Titelseite

Verfahren zur Herstellung von Imidazolidonderivaten und von Biotin, sowie neue Ausgangs- und Zwischenprodukte in diesem Verfahren.

Die vorliegende Erfindung betrifft ein Verfahren zur Herstellung von Imidazolidonderivaten, und zwar von solchen Imidazolidonderivaten, welche als Zwischenprodukte zur Herstellung von Biotin geeignet sind, sowie ein Verfahren zur Herstellung von Biotin selbst. Die Erfindung betrifft ferner neue Ausgangs- und Zwischenprodukte in diesem Verfahren.

Das erfindungsgemässe Verfahren ist dadurch gekennzeichnet, dass man eine Verbindung der allgemeinen Formel

Cot/28.1.80

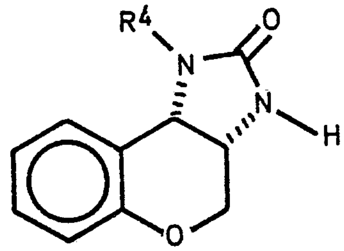

I

worin $R^4$ Wasserstoff bedeutet, nach Ueberführung in ein Alkalimetallsalz, insbesondere das Lithiumsalz, selektiv reduziert und, gewünschtenfalls, an den Stickstoffatomen Schutzgruppen einführt, dass man, gewünschtenfalls, eine so erhaltene Verbindung der allgemeinen Formel

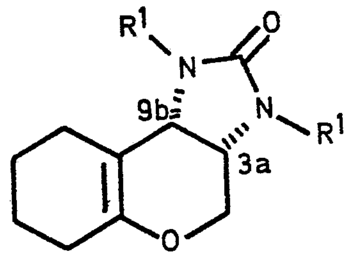

II

worin $R^1$ Wasserstoff oder eine Schutzgruppe bedeutet, oxidiert, dass man, gewünschtenfalls, eine so erhaltene Verbindung der allgemeinen Formel

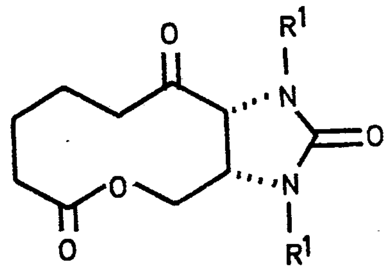

III

worin $R^1$ die obige Bedeutung hat, reduziert, dass man, gewünschtenfalls, eine so erhaltene Verbindung der allgemeinen Formel

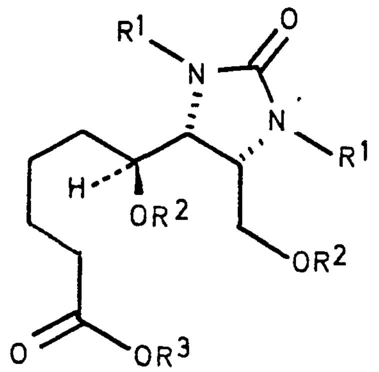

IV

worin $R^1$ die obige Bedeutung hat, $R^2$ Wasserstoff und $R^3$ niederes Alkyl oder Aralkyl bedeutet,

in eine Verbindung der Formel IV überführt, worin $R^2$ eine Organosulfonylgruppe bedeutet, dass man, gewünschtenfalls, letztere Verbindung durch Behandlung mit einem Sulfid in einen Biotinester der allgemeinen Formel

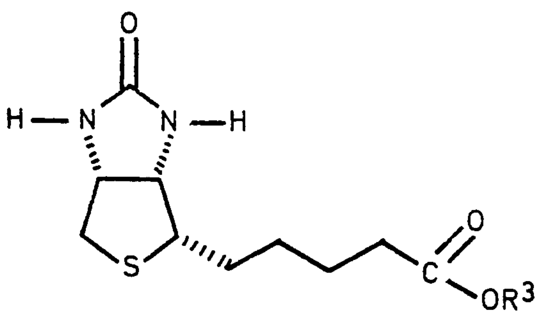

V

worin $R^3$ die obige Bedeutung hat,

überführt und dass man, gewünschtenfalls, einen solchen Biotinester durch Hydrolyse in Biotin überführt.

Bei den vorhergehend genannten Schutzgruppen handelt es sich um in der Biotinchemie als Stickstoff-Schutzgruppen üblicherweise verwendete Schutzgruppen, wie beispielsweise Aralkylgruppen, insbesondere die Benzylgruppe, sowie die Allylgruppe und dergleichen.

Unter "niederen Alkylgruppen" sind im Rahmen der vorliegenden Erfindung solche mit 1-6 C-Atomen zu verstehen, beispielsweise Methyl, Aethyl, Propyl, Isopropyl, Butyl und dergleichen, insbesondere die Methylgruppe.

Unter dem Begriff Organosulfongruppen sind insbesondere die Methansulfongruppe, die Toluolsulfongruppe und die Benzolsulfongruppe zu verstehen.

Die Ueberführung der Verbindung der Formel I in ein Alkalimetallsalz, insbesondere das Lithiumsalz, kann mittels eines Amids erfolgen, beispielsweise mittels Lithiumdiisopropylamid, wobei man zweckmässig in einem Niederalkylamin, beispielsweise in Aethylamin als Lösungsmittel arbeitet und wobei die Reaktiontemperatur je nach dem Siedepunkt des Amins gewählt wird.

Die anschliessende selektive Reduktion wird zweckmässig mit Lithium in einem niederen Alkylamin durchgeführt, und zwar in einem Monoalkylamin oder einem Dialkylamin. Bevorzugt sind hierbei Aethylamin und Dimethylamin, bzw. Gemische hiervon.

Die Reduktion erfolgt hierbei zweckmässig unter Erhitzen am Rückfluss (beim Siedepunkt des Amins), wobei Reaktionszeiten von mehreren Stunden, beispielsweise 14 Stunden, erforderlich sind.

Alternativ zu der oben beschriebenen einstufigen Reduktion kann die Reduktion der Verbindung der Formel I aber auch zweistufig erfolgen. Auch hierbei führt man zuerst die Verbindung der Formel I in ein Alkalimetallsalz, beispielsweise mittels Kalium-ter.-butoxid in das Kaliumsalz, über.

In der ersten Stufe dieser zweistufigen Reduktion wird mit Lithium in flüssigem Ammoniak gearbeitet, wobei man zur entsprechenden Octahydroverbindung gelangt.

In der zweiten Stufe wird dann die Octahydroverbindung katalytisch zur Decahydroverbindung der Formel II hydriert, worin $R^1$ Wasserstoff bedeutet.

In die durch einstufige oder zweistufige Reduktion der Verbindung der Formel I erhaltene Verbindung der Formel II, worin $R^1$ Wasserstoff bedeutet, kann gewünschtenfalls eine Schutzgruppe $R^1$, beispielsweise eine Aralkylgruppe, z.B. die Benzylgruppe, oder die Allylgruppe eingeführt werden. Dies kann in an sich bekannter Weise durch Umsetzung der Verbindung der Formel II mit einem entsprechenden Aralkylhalogenid oder einem Allylhalogenid erfolgen.

Die Einführung einer Schutzgruppe kann jedoch auch an einer späteren Stelle des Reaktionsablaufes, nämlich bei den Verbindungen III oder IV erfolgen.

Die Oxidation einer Verbindung der Formel II zu einer Verbindung der Formel III kann entweder direkt, d.h. einstufig, oder in zwei Stufen durchgeführt werden.

Im Falle der direkten Oxidation kann als Oxidationsmittel beispielsweise Chromsäure in Aceton verwendet werden.

Die direkte Oxidation kann auch mittels einer organischen Persäure durchgeführt werden, beispielsweise mittels Peressigsäure, Perbenzoesäure, halogenierten Perbenzoesäuren, beispielsweise m-Chlorperbenzoesäure, Trifluormethylperbenzoesäure oder Monoperphthalsäure. Bei Verwendung dieser Persäuren sind diese zweckmässig in Mengen von mehreren Aequivalenten einzusetzen.

Eine direkte Oxidation einer Verbindung der Formel II kann auch mittels Oxiden, beispielsweise mittels Rutheniumdioxid, erfolgen.

Bei der zweistufigen Oxidation wird zuerst die der Formel II entsprechende Dihydroxyverbindung gebildet, und es wird dann in der zweiten Stufe diese Dihydroxyverbindung weiter oxidiert zur Verbindung der Formel III.

Die erste Oxidationsstufe kann hierbei ebenfalls wieder mit Persäuren durchgeführt werden, wobei jedoch diese hier zweckmässig in äquivalenten Mengen anzuwenden sind.

Die zweite Oxidationsstufe kann dann mittels Natriumperiodat, gegebenenfalls ohne Isolierung der intermediär gebildeten Dihydroxyverbindung, durchgeführt werden. An Stelle von Natriumperjodat kann aber auch beispielsweise Bleitetraacetat als Oxidationsmittel verwendet werden.

Die Reduktion einer Verbindung der Formel III zu einer Verbindung der Formel IV, worin $R^1$ Wasserstoff oder eine Schutzgruppe und $R^2$ Wasserstoff bedeutet, kann mit einem Metallborhydrid, beispielsweise mit einem Alkalimetallborhydrid, wie Natriumborhydrid, Kaliumborhydrid oder Lithiumborhydrid, oder mit Zinkborhydrid durchgeführt werden. Das bevorzugte Reduktionsmittel ist Natriumborhydrid.

Die Reduktion wird hierbei in Gegenwart eines zu einer Esterbildung befähigten organischen Lösungsmittels durchgeführt, wodurch der Substituent $R^3$ in Formel IV eingeführt wird. Bei diesem Substituenten handelt es sich um eine niedere Alkylgruppe oder um eine Aralkylgruppe, insbesondere die Benzylgruppe, und es wird dementsprechend als Lösungsmittel für die Reduktionsreaktion ein niederes Alkanol, insbesondere Methanol, oder Benzylalkohol verwendet.

Die Reduktion kann bei Raumtemperatur oder bei etwas erhöhten Temperaturen durchgeführt werden; bei Verwendung von Zinkborhydrid sind erhöhte Temperaturen zweckmässig.

Die so erhaltene Verbindung der Formel IV, worin $R^2$ Wasserstoff bedeutet, kann nun in eine Verbindung der Formel IV übergeführt werden, worin $R^2$ eine Organosulfonylgruppe bedeutet. Diese Ueberführung kann mittels eines geeigneten Sulfonsäurehalogenids, beispielsweise mittels Methansulfonylchlorid, Toluolsulfonylchlorid oder Benzolsulfonylchlorid erfolgen. Als Lösungsmittel für diese Reaktion wird zweckmässig eine tertiäre Base verwendet, beispielsweise Pyridin. Die Umsetzung kann bei Temperaturen zwischen etwa -10°C und Raumtemperatur erfolgen.

Die Ueberführung einer Verbindung der Formel IV, worin $R^2$ eine Organosulfonylgruppe darstellt, in eine Verbindung der Formel V, kann durch Behandlung mit einem Sulfid erfolgen. Als Sulfid verwendet man hierbei zweckmässig ein Alkalimetall- oder Erdalkalimetallsulfid, insbesondere Natriumsulfid.

Die Umsetzung wird zweckmässig in Dimethylformamid oder Hexamethylenphosphorsäuretriamid als Lösungsmittel durchgeführt. Zweckmässig wird das Sulfid bei Raumtemperatur zugesetzt und hierauf auf etwa 100°C erhitzt.

Nach Destillation des Lösungsmittels kann das rohe Reaktionsgemisch direkt der Hydrolyse unterworfen werden, wobei aus dem Biotinester der Formel V die freie Säure freigesetzt wird. Die Hydrolyse erfolgt in üblicher Weise mit einem Alkali, beispielsweise mit Natronlauge.

Die in dem erfindungsgemässen Verfahren als Ausgangsmaterial verwendeten Verbindungen der Formel I, sowie die Produkte der Formeln II und III sind neue Verbindungen und als solche ebenfalls Gegenstand der vorliegenden Erfindung.

Die neuen Verbindungen der Formel I, worin $R^4$ Wasserstoff oder auch die Azidosulfonylgruppe darstellt, können gemäss folgendem Reaktionsschema aus 2H-Chromen der Formel VI über die Verbindungen der Formeln VII und VIII hergestellt werden.

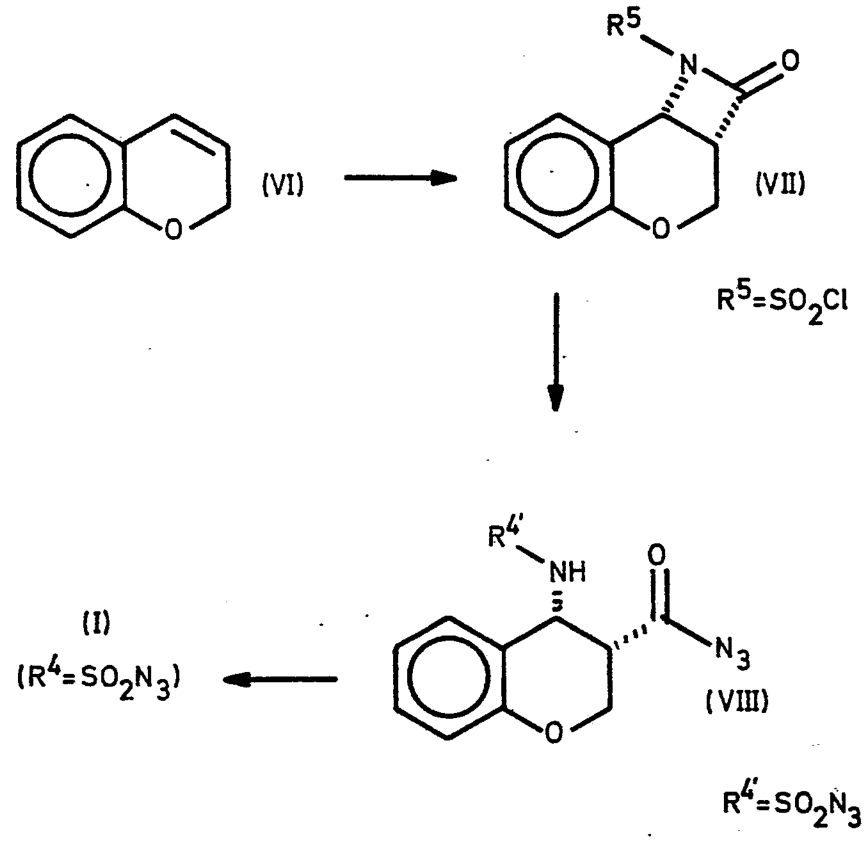

(VI) (VII)

$R^5=SO_2Cl$

(I)
$(R^4=SO_2N_3)$

(VIII)

$R^{4'}=SO_2N_3$

Hierbei wird in der ersten Stufe das 2H-Chromen der Formel VI mit Chlorsulfonylisocyanat zur Verbindung der Formel VII umgesetzt, worin $R^5$ die Gruppe $-SO_2Cl$ bedeutet.

Die Umsetzung erfolgt zweckmässig in einem inerten organischen Lösungsmittel, beispielsweise einem Chlorkohlenwasserstoff, wie Methylenchlorid oder Chloroform, oder in einem Aether, wie Tetrahydrofuran und dergleichen.

Die Umsetzung wird zu Beginn bei möglichst tiefen Temperaturen, nämlich bei Temperaturen zwischen -60°C und -35°C durchgeführt, wobei man beispielsweise 3 Stunden bei -60°C stehen lässt und dann innerhalb von 4 Stunden auf -35°C erwärmen lässt und bei dieser Temperatur über Nacht

stehen lässt. Man erhält hierbei die Verbindung der Formel VII, worin $R^5$ die Gruppe $-SO_2Cl$ bedeutet.

Diese Verbindung wird nun in einem weiteren Schritt im System Natriumazid/Triäthylammoniumazid/Stickstoffwasserstoffsäure, in einem inerten organischen Lösungsmittel, beispielsweise einem halogenierten Kohlenwasserstoff, wie Methylenchlorid oder Chloroform, bei tiefen Temperaturen, vorzugsweise bei Temperaturen von etwa -10°C bis etwa -20°C, in die Verbindung der Formel VIII übergeführt, worin $R^{4'}$ die Azidosulfonylgruppe bedeutet.

Diese Verbindung wird mittels Curtius-Abbau, beispielsweise durch Erhitzen in einem organischen Lösungsmittel, beispielsweise in Toluol oder Benzol, auf etwa 60°-100°C, vorzugsweise auf etwa 80°C, in die Verbindung der Formel I übergeführt, worin $R^4$ die Azidosulfonylgruppe bedeutet.

Diese letztere Verbindung kann dann durch Abspaltung der Azidosulfonylgruppe in die Verbindung der Formel I, worin $R^4$ Wasserstoff bedeutet, übergeführt werden.

Diese Abspaltung kann in einfacher Weise dadurch erfolgen, dass man die Verbindungen der Formel I, worin $R^4$ die Azidosulfonylgruppe bedeutet, mit einem Alkalimetallsulfit, beispielsweise Natriumsulfit, in wässriger Lösung zum Sieden erhitzt. An Stelle des Sulfits kann auch Schwefelwasserstoff verwendet werden.

Die Verbindungen der Formeln VII und VIII sind ebenfalls neu und als solche ebenfalls Gegenstand der vorliegenden Erfindung.

Beispiel 1

a) 5,71 g 3a,9b-cis-1,2,3,4a,4,9b-hexahydro-chromeno[3,4-d]imidazol-2-on und 1,38 g Lithiumamid werden in einem 200 ml Rundkolben in 45 ml Aethylamin suspendiert. Der Rundkolben wird über einen Anschützaufsatz an einen Dewarkühler, der über einen Kryostaten auf -50°C gekühlt wird, angeschlossen.

Die Apparatur wird mit über Kaliumhydroxid getrocknetem Stickstoff gespült und die Suspension 4 Stunden unter Rückfluss magnetisch gerührt. Danach gibt man durch den Anschützaufsatz 1,67 g Lithium (3 mm-Draht), spült kurz mit Stickstoff und rührt 10 Minuten. Anschliessend werden in 10-minütigen Zeitabständen unter Stickstoff, ohne das Rühren zu unterbrechen, je 10 ml, 10 ml, 30 ml, 30 ml Dimethylamin zugegeben. Das Reaktionsgemisch wird 14 Stunden gerührt, dann, um das tiefblaue Reaktionsgemisch zu entfärben und um nicht umgesetztes Lithium zu entfernen, durch einen mit Glaswolle gepackten Filter filtriert. Nach Abziehen der Amine (Stickstoffatmosphäre) wird der gelbliche Rückstand mit 100 g Eis versetzt und eine halbe Stunde gerührt. Nach Filtrieren des ausgefallenen Reaktionsproduktes, Einengen und Sättigen der Mutterlauge mit Ammonsulfat und erneuter Filtration wird das gesamte Filtergut 3 x mit 10 ml kaltem Wasser gewaschen, getrocknet und aus 200 ml Methanol umkristallisiert. Nach Einengen der Mutterlauge und Trocknen (24 Stunden bei 80°C und 0,01 mm Hg) werden 3,7 g 3a,9b-cis-1,2,3,3a,4,6,7,8,9,9b-Decahydro-chromeno[3,4-d]imidazol-2-on erhalten; Smp.: 257-260°C.

b) 3,94 g der so erhaltenen Substanz werden in einem 250 ml Rundkolben in 70 ml Wasser, 3 ml Acetonitril und 5 ml Aether suspendiert. Der erhaltenen Suspension werden unter starkem Rühren langsam (während 2 Stunden) 4,1 g m-Chlorperbenzoesäure in 50 ml Aether bei Raumtemperatur zugetropft. Nach beendeter Zugabe trennt man die Phasen im

Scheidetrichter, extrahiert die wässrige Phase 3 x mit 30 ml Aether, die vereinigten Aetherphasen 1 x mit 5 ml Wasser und befreit anschliessend die wässrige Phase am Rotationsverdampfer von Lösungsmittelresten. Diese wässrige Lösung wird auf 0°C gekühlt und eine eiskalte Lösung von 4,33 g Natriumperjodat in 40 ml Wasser zugesetzt. Man lässt 2 Stunden bei 0°C stehen, erwärmt auf Raumtemperatur und hält 2 Stunden bei dieser Temperatur. Nach Abfiltrieren des auskristallisierten Reaktionsproduktes wird das Filtrat auf 40 ml eingeengt. Nach erneuter Filtration wird das gesamte Reaktionsprodukt aus Wasser umkristallisiert. Einschliesslich des nach Einengen der Mutterlauge erhaltenen Produktes erhält man nach Trocknen über Phosphorpentoxid (10 Stunden bei 60°C und 0,01 mm Hg) 3,65 g 1,10-cis-3-Oxa-11,13-diaza-bicyclo[8,3,0]tridecan-4,9,12-trion; Smp. 215-230°C (Zersetzung).

c) 1 g feingepulvertes 1,10-cis-3-Oxa-11,13-diaza-bicyclo[8.3.0]-tridecan-4,9,12-trion wird in 60 ml absolutem Methanol mit 0,24 g Natriumborhydrid in Gegenwart von 0,7 g Molekularsieb 3Å zwei Stunden bei Raumtemperatur gerührt und dann 7 Stunden stehengelassen. Nach Filtration wird der pH-Wert der Reaktionslösung mit Eisessig auf 4,7 gebracht und das Lösungsmittel abdestilliert. Der Rückstand wird 3 x mit je 15 ml absolutem Methanol versetzt und das Methanol jeweils wieder abgedampft und anschliessend wird der Rückstand mit 20 ml absolutem Methanol versetzt. Nach Abdestillieren des Methanols bei Normaldruck wird am Rotationsverdampfer von Lösungsmittelresten befreit. Der Rückstand wird mit 5 ml gesättigter Ammonsulfatlösung versetzt, worauf Kristallisation eintritt. Das erhaltene Produkt wird abfiltriert, getrocknet und aus wenig Methanol umkristallisiert. Man erhält 0,73 g eines Gemisches von (εR,4S,5S) und (εS,4R,5R)-5-Hydroxymethyl-2-oxo-ε-hydroxy-4-imidazolidincapronsäuremethylester mit einem Schmelzpunkt von 165-167°C (aus Methanol).

d) 0,3 g dieses rohen Gemisches werden in 5 ml absolutem Pyridin gelöst, worauf auf -10°C gekühlt wird. Unter Rühren und unter Feuchtigkeitsausschluss tropft man zu der erhaltenen Lösung 1,32 g Methansulfonsäurechlorid, hält die Temperatur noch 10 Minuten bei -10°C und rührt anschliessend 2 Stunden bei Raumtemperatur weiter. Ein Grossteil des Pyridins wird dann bei Raumtemperatur unter einem Druck von 0,05 mm Hg abdestilliert und der Rückstand mit 10 ml Petroläther (Sdp- 40-50°C) verrührt. Die Petrolätherphase wird abdekantiert und der Rückstand bei einem Druck von 0,05 mm Hg vom Petrolätherrest befreit. Nach Zugabe von 5 g Eis wird 3 x mit 10 ml Methylenchlorid extrahiert. Die vereinigten Methylenchloridextrakte werden, nachdem sie 3 x mit je 3 ml 2N Salzsäure und 1x mit 5 ml Eiswasser gewaschen wurden, über Natriumsulfat getrocknet. Nach Filtrieren wird das Methylenchlorid am Rotationsverdampfer abdestilliert und der Rückstand bei vermindertem Druck unter Raumtemperatur getrocknet.

e) Das auf diese Weise erhaltene rohe Gemisch von (εR,4S,5S)-und (εS,4R,5R)-Methylensulfonyloxymethyl-2-oxo-ε-methylsulfonyloxy-4-methazolidincapronsäuremethylester (0,34 g) wird in 5 ml Hexamethylenphosphorsäuretriamid gelöst und die Lösung mit Stickstoff entgast. Zu der Lösung werden bei Raumtemperatur unter Rühren 0,21 g Natriumsulfid ($Na_2S.9H_2O$) zugesetzt und die Lösung wird unter Stickstoff innerhalb von 30 Minuten auf 100°C erhitzt. Nach zweistündigem Rühren bei 100°C wird der Grossteil des Hexamethylenphosphorsäuretriamids am Wasserbad bei 0,01 mm Hg abdestilliert. Der gelbe Rückstand wird mit 20 ml Petroläther (Sdp. 40-50°C) verrührt und die Petrolätherphase abdekantiert. Nach dem Entfernen von Petrolätherresten am Rotationsverdampfer nimmt man mit 10 ml 2N Natronlauge auf, versetzt mit 0,2 g Aktivkohle und erhitzt 3 Stunden am siedenden Wasserbad. Die heisse Lösung wird filtriert, das Filtrat mit konzentrierter Salzsäure auf einen pH-Wert von 3 gebracht, auf 3 ml eingeengt und 30

Minuten auf $0^{o}C$ gekühlt. Die überstehende Lösung wird vom ausgefallenen Produkt abdekantiert, und letzteres erneut mit 10 ml Wasser und 0,1 g Aktivkohle 15 Minuten unter Rückfluss erhitzt. Nach Filtrieren wird auf 3 ml eingeengt und 1 Stunde im Eisbad gekühlt. Das so erhaltene flockige, gelbliche Produkt wird abfiltriert, trocken gepresst und in 3 ml siedendem Methanol aufgenommen. Nach Verreiben und Filtration wird das so erhaltene nahezu farblose Produkt aus wenig Wasser umkristallisiert. Nach dem Trocknen über Phosphorpentoxid (bei $80^{o}C$, 0,05 mm Hg, während 14 Stunden erhält man farblose, nadelige Kristalle von ($\pm$)Biotin mit einem Schmelzpunkt 236-$237^{o}C$.

Der Methyläther dieser Verbindung schmilzt bei 132-$134^{o}C$.

## Beispiel 2

Die Reduktion von 3a,9b-cis-1,2,3,4a,4,9b-Hexahydro-chromeno[3,4-d]imidazol-2-on zum 3a,9b-cis-1,2,3,3a,4,6,7,8,9,9b-Decahydro-chromeno[3,4-d]imidazol-2-on kann, statt wie in Beispiel 1a) beschrieben einstufig, auch in zwei Stufen durchgeführt werden, wobei man in der ersten Stufe zur entsprechenden Octahydroverbindung gelangt. Es wird hierbei wie folgt vorgegangen:

Eine Suspension von 2 g 3a,9b-cis-1,2,3,4a,4,9b-Hexahydro-chromeno[3,4-d]imidazol-2-on in 150 ml flüssigem Ammoniak wird mit 6 g Kalium-tert.-Butoxid versetzt und eine halbe Stunde gerührt. Nach Zugabe von 30 ml tert.-Butanol wird im Verlauf von 60 Minuten allmählich 0,8 g Lithium, das in 25 mg Portionen zerschnitten wurde, eingetragen. Die Zugabe des Lithium soll so schnell erfolgen, dass während der gesamten Reaktionszeit eine blaue Färbung des Reaktionsgemisches zu beobachten ist. Sobald nach beendeter Zugabe des Lithium die blaue Farbe des Reaktionsgemisches verschwunden ist, werden 150 ml absolutes Methanol zugegeben,

um das vorhandene tert.-Butoxid zu zersetzen. Nach Abdestillation des Ammoniak werden dem methanolischen Rückstand 100 g Eis zugesetzt und der Methylalkohol am Rotationsverdampfer zur Gänze abdestilliert. Das aus der auf 0°C abgekühlten Reaktionslösung ausgefallene Produkt wird nach Filtrieren 3 x am Filter mit 25 ml Eiswasser gewaschen. Nach dem Trocknen wird das Rohprodukt aus wenig Acetonitril, dem 0,25 g Maleinsäureanhydrid zugefügt wurden, umkristallisiert. Nach dem Trocknen (14 Stunden bei 25°C und 0,01 mm Hg) erhält man 1,4 g 3a,9b-cis-1,2,3,3a,4,6,9,9b-Octahydro-chromeno[3,4-d]-imidazol-2-on, welches nach Umkristallisation aus Methanol bei 253-255°C schmilzt.

Zu 20 mg Platinoxid in 5 ml absolutem Methanol werden 0,1 g der nach den obigen Angaben erhaltenen Octahydroverbindung, gelöst in 22 ml absolutem Methanol, bei 20°C in einer Wasserstoffatmosphäre (770 mm Hg) gegeben. Nach Einschalten des Rührwerkes wird innerhalb von 10 Minuten die berechnete Menge Wasserstoff aufgenommen. Nach 15 Minuten wird abgebrochen, vom Katalysator abfiltriert und das Lösungsmittel am Rotationsverdampfer entfernt. Der kristalline Rückstand, die entsprechende Decahydroverbindung, schmilzt bei 250-253°C.

Beispiel 3

Das in den Beispielen 1 und 2 als Ausgangsmaterial verwendete 3a,9b-cis-1,2,3,4a,4,9b-Hexahydrochromeno[3,4-d] imidazol-2-on, kann wie folgt hergestellt werden:

14,2 g Chlorsulfonylisocyanat werden in einem 100 ml Rundkolben, ausgerüstet mit einem mit Calciumchlorid und Phosphorpentoxid beschickten Trockenrohr, mit flüssigem Stickstoff gekühlt, bis das Chlorsulfonylisocyanat erstarrt. Hierauf setzt man rasch 13,1 g 2H-Chromen und 6,5 ml absoluten Aether zu, wobei eine leicht rote Verfärbung eintritt. Hierauf wird sofort 10 Minuten mit flüssigem Stickstoff

gekühlt. Anschliessend lässt man das Reaktionsgefäss 3 Stunden bei -60°C stehen, erwärmt danach innerhalb 4 Stunden auf -35°C und lässt über Nacht (16 Stunden) bei -35°C stehen. Hierauf erwärmt man 4 Stunden auf -25°C und kühlt erneut auf -35°C ab, wodurch der Kolbeninhalt zu einer leicht gelblich-rot verfärbten Kristallmasse erstarrt, welche aus rohem 1-Chlorsulfonyl-2,3,4,10-tetrahydro-1H-chromeno[3,4-d]azet-2-on besteht.

Aus dieser Substanz kann durch reduktive Entfernung der Sulfochloridfunktion mittels Natriumjodid und Natriumbicarbonat die Verbindung der Formel VII erhalten werden, worin $R^5$ Wasserstoff bedeutet. Diese schmilzt bei 196-197,5°C (aus Chloroform).

Für die weitere Verarbeitung des nach den obigen Angaben erhaltenen N-Chlorsulfonyl-β-lactam ist ein Puffersystem erforderlich, welches wie folgt zubereitet wird:

Einer unter Rühren hergestellten Suspension von 27,3g Natriumazid, 27,6 g Wasser und 80 ml Methylenchlorid werden nach dem Abkühlen auf 0°C 20,58 g konzentrierte Schwefelsäure so langsam zugetropft, dass die Reaktionstemperatur nicht über +10°C ansteigt. Nach beendeter Zugabe rührt man noch 20 Minuten bei 0°C, dekantiert vom ausgefallenen Natriumsulfat und wäscht den Rückstand 4 x mit je 10 ml Methylenchlorid. In die vereinigten, mit Natriumsulfat getrockneten und durch Aussenkühlung auf -20°C abgekühlten Dekantate werden 21,5 g Triäthylamin unter Rühren eingetragen, wobei die Innentemperatur nicht über -8°C steigen soll.

Zum rohem, nach den obigen Angaben erhaltenen 1-Chlorsulfonyl-2,3,4,10-tetrahydro-1H-chromeno[3,4-c]azet-2-on(N-chlorsulfonyl-β-lactam) werden 50 ml absolutes Methylenchlorid zugesetzt und bei -10°C eine möglichst grosse Menge des Lactams gelöst. Die so erhaltene Lösung

wird zu dem bei -20°C gehaltenen, nach den obigen Angaben bereiteten Puffersystem so zugetropft, dass die Temperatur zwischen -18°C und -16°C bleibt. Nach Zusatz dieser 50 ml gibt man 30 ml Methylenchlorid zum noch ungelösten Lactam, löst dieses bei 0°C und tropft die erhaltene Lösung wieder dem Puffersystem zu. Nach beendeter Zugabe wird innerhalb von 30 Minuten unter Rühren auf Raumtemperatur erwärmt und dann mit 100 ml Eiswasser versetzt. Hierauf wird unter starkem Rühren der pH-Wert der wässrigen Phase mit konzentrierter Salzsäure auf 3,5 gebracht. Nach Trennung der Phasen wäscht man die Methylenchlorid-Phase 3 x mit 50 ml kaltem Wasser. Nach dem Trocknen über Natriumsulfat und Filtrieren wird das Methylenchlorid bei Raumtemperatur am Rotationsverdampfer abdestilliert. Das so erhaltene Bisazid der Formel VIII, worin $R^{4'}$ die Azidosulfongruppe bedeutet, ist eine gelbliche, semi-kristalline bis ölige Substanz. Sie wird mit 200 ml absolutem Toluol aufgenommen und am Wasserbad auf 85°C erhitzt. Es setzt Stickstoffentwicklung ein und nach einiger Zeit beginnt sich ein voluminöser nadeliger Niederschlag auszuscheiden. Man erhitzt hierauf weitere 15 Minuten unter Schütteln am siedenden Wasserbad, kühlt anschliessend auf Raumtemperatur ab und lässt eine halbe Stunde stehen. Der Niederschlag wird abfiltriert und am Filter 4 x mit 10 ml eiskaltem Methanol gewaschen. Nach dem Trocknen (3 Stunden bei 60°C und 0,1 mm Hg ) erhält man 16,5 g 3a,9b-cis 1-Azidosulfonyl-1,2,3,3a,4,9b-hexahydrochromeno[3,4-d]imidazol-2-on in Form von farblosen Nadeln mit einem Schmelzpunkt von 195°C. Nach Umkristallisation aus Methanol schmilzt die Substanz bei 191-192°C.

27,2 g der nach den obigen Angaben erhaltenen Azidosulfonylverbindung, 23,2 g Natriumsulfit und 500 ml Wasser werden in einem 4 l Kolben 35 Minuten unter Schütteln unter Rückfluss gekocht. Beim Abkühlen setzt Kristallisation ein, die bei 0°C vervollständigt wird. Der Niederschlag wird abfiltriert und mit 50 ml kaltem Wasser gewaschen. Weiteres Reaktionsprodukt wird durch Einengen der Mutterlauge auf

80 ml, analoge Kristallisation und Waschen mit Wasser gewonnen. Nach dem Trocknen über Phosphorpentoxid (4 Stunden bei 80°C und 0,1 mm Hg) erhält man 17,3 g 3a,9b-cis 1,2,3,4a,4,9b-hexahydro-chromeno[3,4-d]imidazol-2-on mit einem Schmelzpunkt von 265°C (aus Wasser). Nach Umkristallisation aus Aethanol schmilzt die Substanz bei 260-263°C.

## Patentansprüche

1) Verfahren zur Herstellung von Imidazolidonderivaten, sowie von Biotin, dadurch gekennzeichnet, dass man eine Verbindung der allgemeinen Formel

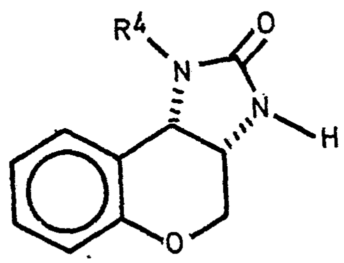

I

worin $R^4$ Wasserstoff bedeutet, nach Ueberführung in ein Alkalimetallsalz, insbesondere das Lithiumsalz, selektiv reduziert und, gewünschtenfalls, an den Stickstoffatomen Schutzgruppen einführt, dass man, gewünschtenfalls, eine so erhaltene Verbindung der allgemeinen Formel

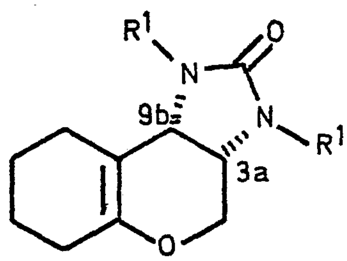

II

worin $R^1$ Wasserstoff oder eine Schutzgruppe bedeutet, oxidiert, dass man, gewünschtenfalls, eine so erhaltene Verbindung er allgemeinen Formel

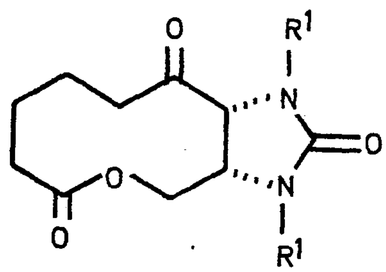

III

worin $R^1$ die obige Bedeutung hat, reduziert, dass man, gewünschtenfalls, eine so erhaltene Verbindung der allgemeinen Formel

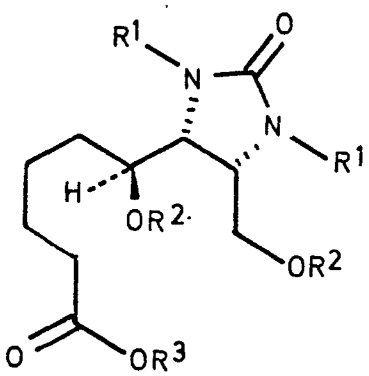

IV

worin $R^1$ die obige Bedeutung hat, $R^2$ Wasserstoff und $R^3$ niederes Alkyl oder Aralkyl bedeuten, in eine Verbindung der Formel IV überführt, worin $R^2$ eine Organosulfonylgruppe bedeutet, dass man, gewünschtenfalls, letztere Verbindung durch Behandlung mit einem Sulfid in einen Biotinester der allgemeinen Formel

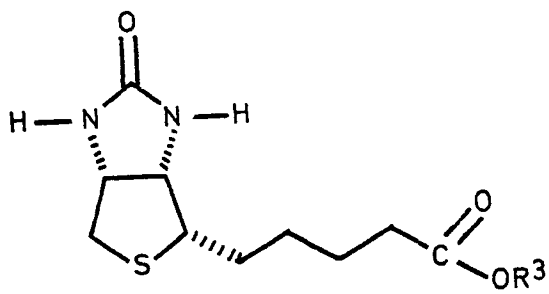

V

worin $R^3$ die obige Bedeutung hat, überführt und dass man, gewünschtenfalls, einen solchen Biotinester durch Hydrolyse in Biotin überführt.

2) Verfahren nach Anspruch 1, dadurch gekennzeichnet, dass man die Reduktion der Verbindung der Formel I als Lithium/Amin-Reduktion durchführt.

3) Verfahren nach Anspruch 1, dadurch gekennzeichnet, dass man die Reduktion der Verbindung der Formel I zweistufig durchführt, wobei man in der ersten Stufe mittels einer Metall/Amin-Reduktion, insbesondere mittels Lithium in flüssigem Ammoniak, zu einer Octahydroverbindung und in der zweiten Stufe katalytisch zur Decahydroverbindung reduziert.

4) Verfahren nach Anspruch 1, dadurch gekennzeichnet, dass man die Oxydation einer Verbindung der Formel II mittels Chromsäure oder einer organischen Persäure durchführt.

5) Verfahren nach Anspruch 1, dadurch gekennzeichnet, dass man die Reduktion einer Verbindung der Formel III mit einem Metallborhydrid, beispielsweise mit einem Alkalimetallborhydrid, insbesondere Natriumborhydrid, oder mit Zinkborhydrid durchführt.

6) Verfahren nach Anspruch 1, dadurch gekennzeichnet, dass man eine Verbindung der Formel IV, worin $R^2$ Wasserstoff bedeutet, in eine Verbindung der Formel IV überführt, worin $R^2$ die Methansulfongruppe, die Toluolsulfongruppe oder die Benzolsulfongruppe bedeutet.

7) Verfahren nach Anspruch 1, dadurch gekennzeichnet, dass man die Verbindung der Formel IV, worin $R^2$ eine Organosulfongruppe bedeutet, durch Behandlung mit einem Alkalimetallsulfid, insbesondere mit Natriumsulfid und anschliessende Hydrolyse des erhaltenen Biotinesters in Biotin überführt.

8) Verbindungen der allgemeinen Formel

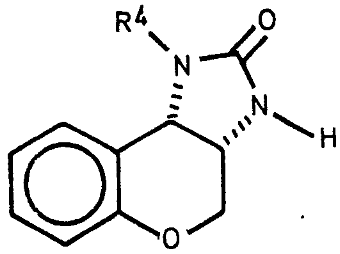

I

worin $R^4$ Wasserstoff oder Azidosulfonyl bedeutet.

9) Verbindungen der allgemeinen Formel

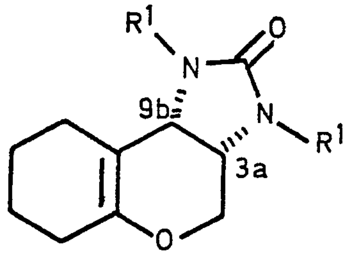

II

worin $R^1$ Wasserstoff oder eine Schutzgruppe bedeutet.

10) Verbindungen der allgemeinen Formel

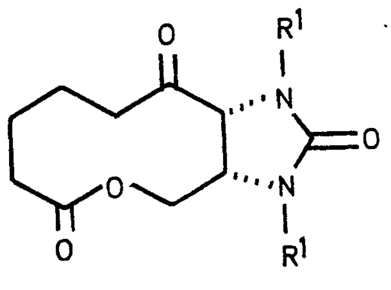

III

worin $R^1$ Wasserstoff oder eine Schutzgruppe bedeutet.

11) Verbindung der Formel

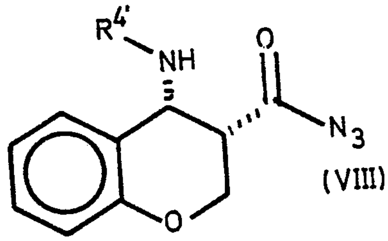

(VIII)

worin $R^{4'}$ Azidosulfonyl bedeutet.

12) Verbindungen der allgemeinen Formel

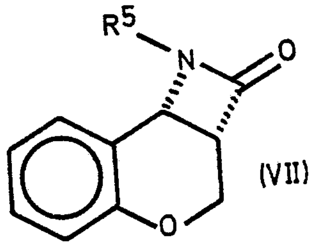

(VII)

worin $R^5$ Wasserstoff oder $-SO_2Cl$ bedeutet.

*****

Patentansprüche für "Oesterreich"

1) Verfahren zur Herstellung von Imidazolidonderivaten, sowie von Biotin, dadurch gekennzeichnet, dass man eine Verbindung der allgemeinen Formel

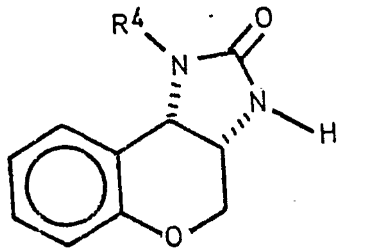

I

worin $R^4$ Wasserstoff bedeutet, nach Ueberführung in ein Alkalimetallsalz, insbesondere das Lithiumsalz, selektiv reduziert und, gewünschtenfalls, an den Stickstoffatomen Schutzgruppen einführt, dass man, gewünschtenfalls, eine so erhaltene Verbindung der allgemeinen Formel

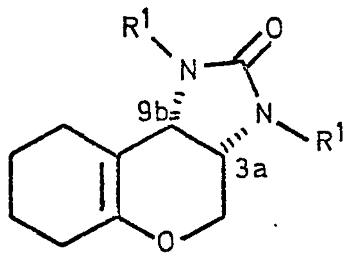

II

worin $R^1$ Wasserstoff oder eine Schutzgruppe bedeutet, oxidiert, dass man, gewünschtenfalls, eine so erhaltene Verbindung er allgemeinen Formel

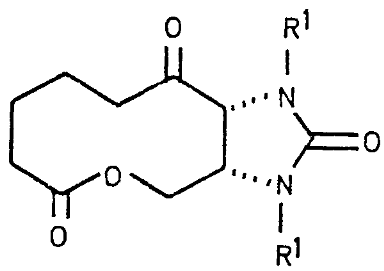

III

0022446
Österreich

worin $R^1$ die obige Bedeutung hat, reduziert, dass man, gewünschtenfalls, eine so erhaltene Verbindung der allgemeinen Formel

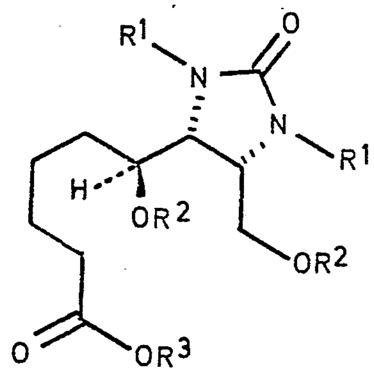

IV

worin $R^1$ die obige Bedeutung hat, $R^2$ Wasserstoff und $R^3$ niederes Alkyl oder Aralkyl bedeuten, in eine Verbindung der Formel IV überführt, worin $R^2$ eine Organosulfonylgruppe bedeutet, dass man, gewünschtenfalls, letztere Verbindung durch Behandlung mit einem Sulfid in einen Biotinester der allgemeinen Formel

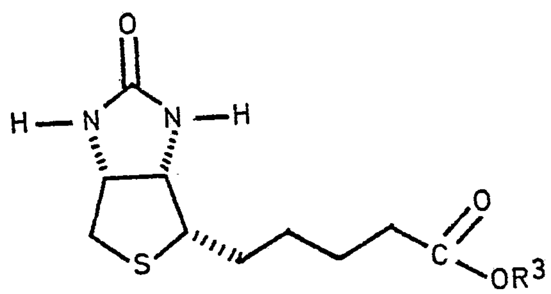

V

worin $R^3$ die obige Bedeutung hat, überführt und dass man, gewünschtenfalls, einen solchen Biotinester durch Hydrolyse in Biotin überführt.

2) Verfahren nach Anspruch 1, dadurch gekennzeichnet, dass man die Reduktion der Verbindung der Formel I als Lithium/Amin-Reduktion durchführt.

3) Verfahren nach Anspruch 1, dadurch gekennzeichnet, dass man die Reduktion der Verbindung der Formel I zweistufig durchführt, wobei man in der ersten Stufe mittels einer Metall/Amin-Reduktion, insbesondere mittels Lithium in flüssigem Ammoniak, zu einer Octahydroverbindung und in der zweiten Stufe katalytisch zur Decahydroverbindung reduziert.

4) Verfahren nach Anspruch 1, dadurch gekennzeichnet, dass man die Oxydation einer Verbindung der Formel II mittels Chromsäure oder einer organischen Persäure durchführt.

5) Verfahren nach Anspruch 1, dadurch gekennzeichnet, dass man die Reduktion einer Verbindung der Formel III mit einem Metallborhydrid, beispielsweise mit einem Alkalimetallborhydrid, insbesondere Natriumborhydrid, oder mit Zinkborhydrid durchführt.

6) Verfahren nach Anspruch 1, dadurch gekennzeichnet, dass man eine Verbindung der Formel IV, worin $R^2$ Wasserstoff bedeutet, in eine Verbindung der Formel IV überführt, worin $R^2$ die Methansulfongruppe, die Toluolsulfongruppe oder die Benzolsulfongruppe bedeutet.

7) Verfahren nach Anspruch 1, dadurch gekennzeichnet, dass man die Verbindung der Formel IV, worin $R^2$ eine Organosulfongruppe bedeutet, durch Behandlung mit einem Alkalimetallsulfid, insbesondere mit Natriumsulfid und anschliessende Hydrolyse des erhaltenen Biotinesters in Biotin überführt.

Europäisches Patentamt

# EUROPÄISCHER RECHERCHENBERICHT

0022446

Nummer der Anmeldung

EP 80 10 1546.2

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A1 - 2 508 831 (AGENCE NATIONALE DE VALORISATION DE LA RECHERCHE) * Ansprüche 1, 7, 9 * -- | 1,6,7 |
| | Patents Abstracts of Japan Band 2, Nr. 110, 13. September 1978, Seite 2081 C 78 & JP - A - 53 - 73589 -- | 1,6,7 |
| | Patents Abstracts of Japan Band 2, Nr. 110, 13. September 1978 Seite 2081 C 78 & JP - A - 53 73588 -- | 1 |
| A | US - A - 4 044 020 (SYNTEX) ---- | |

**KLASSIFIKATION DER ANMELDUNG (Int.Cl.3)**

C 07 D 495/04
C 07 D 491/044
C 07 D 491/052
C 07 D 311/68
//(C 07 D 495/04, 333/00, 235/00)
(C 07 D 491/044, 313/00, 235/00)
(C 07 D 491/052, 311/00, 235/00)
(C 07 D 491/052, 311/00, 205/00)

**RECHERCHIERTE SACHGEBIETE (Int. Cl.3)**

C 07 D 233/32
C 07 D 311/68
C 07 D 491/044
C 07 D 491/052
C 07 D 495/04

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 22-07-1980 | FROELICH |